# EUROPEAN PATENT SPECIFICATION

(11) **EP 3 461 377 B1**
(45) Date of publication and mention of the grant of the patent: **25.11.2020**
(21) Application number: 17194394.7
(22) Date of filing: 02.10.2017
(51) Int. Cl.: A47G 9/02, A01K 13/00

(54) **THERAPEUTIC BLANKET WITH WEIGHT ELEMENTS**
THERAPEUTISCHE DECKE MIT GEWICHTSELEMENTEN
COUVERTURE THÉRAPEUTIQUE AVEC ÉLÉMENTS DE POIDS

(43) Date of publication of application: 03.04.2019
(73) Proprietor: Nilsson, Rasmus, 411 29 Göteborg (SE); Brar, Max, 122 44 Hägersten (SE)
(72) Inventor: Nilsson, Rasmus, 411 29 Göteborg (SE); Brar, Max, 122 44 Hägersten (SE)
(74) Representative: AWA Sweden AB

(56) References cited:
- WO-A1-03/055361
- WO-A1-2014/116163
- WO-A1-2018/013025
- SE-A1- 1 651 725
- US-A1- 2011 047 698

## Description

### FIELD OF THE INVENTION

The present invention relates to a therapeutic blanket with weight elements. It particularly relates to a therapeutic blanket with weight elements being individually fixed at predefined positions in the blanket.

### BACKGROUND OF THE INVENTION

Weighted therapeutic blankets are designed to put more pressure on a user's body than a conventional blanket. Today weighted therapeutic blankets are used as a therapy for a number of conditions and symptoms related to difficulties in finding rest and sleeping. These symptoms may for example be related to hyperactivity, ADHD, Asperger's syndrome, autism, insomnia, lack of body awareness, and other neuropsychical diagnoses. The pressure applied by the weighted blanket helps by increasing the user's awareness of her/his body, which often calms the user.

Weighted therapeutic blankets are not only used by humans, but may also be used by animals. For example, they may be used for trotting horses, to help them relax and focus before a race. Another field of use is for racing dogs, as well as hyperactive dogs, dogs suffering from stress or anxiety, and dogs that are difficult to control in loud environments.

Today there are various different types of weighted therapeutic blankets on the market, see for example WO 03/055361 A1. The main principle of these is that a weight element is sown into channels or pockets, and placed between two layers of textile. The weight element may for example be chains, plastic balls or pellets.

A drawback of using plastic balls or pellets sown into patches which are placed in the blanket is that the blanket becomes thick, which makes it space consuming, warm and difficult to handle. Blankets of this type do not breathe well, which results in that the filling material collects moist, and takes a long time to dry. Using chains sown into channels makes it possible to regulate the thickness of the blanket, and it becomes less space consuming and breathes better. However, chains in channels do not apply an evenly distributed pressure to the user's body. Pressure will only be applied where the chains are located, and only on top of the user's body. This results in that parts of the user's body which may be placed between two chains, such as the legs, may not receive any additional pressure, at least temporarily. The therapeutic effect of the blanket with chains may therefore be at least temporarily reduced. In addition to this, blankets with chains may be noisy, and they also need a sturdy frame of a coarse material in order to hold the chains. This frame reduces user comfort. There is therefore a need for providing a blanket having an improved pressure distribution, both spatially and in time, and hence an improved therapeutic effect. There is also a need for providing a blanket which breathes well and is not too space consuming.

### SUMMARY OF THE INVENTION

It is an object of the present invention to alleviate at least some of the mentioned drawbacks of the prior art and to provide an improved therapeutic blanket with weight elements. This and other objects, which will become apparent in the following, are accomplished by a therapeutic blanket as defined in the accompanying independent claims.

The present invention is at least partially based on the realisation that by individually arranging weight elements at predefined positions within a therapeutic blanket, where none of the weight elements are in direct contact with each other, a blanket with better breathing properties and less noise issues may be achieved. In more detail, by individually fixing and thereby distributing the weight elements across the blanket surface area, the blanket can be made more flexible/adaptable and a better "point pressure" distribution can be provided for, as compared to prior known solutions having a plurality of linked objects arranged in channels or the like.

The term exemplary should in this application be understood as serving as an example, instance or illustration.

According to a first aspect of the present invention, a therapeutic blanket according to claim 1 is provided.

The fixation or fastening of each weight element at a predefined position does mean that the weight elements are arranged such that each weight element has a limited volume or area within which it can move. Stated differently, fixed at a predefined position is arranged within a predefined volume or area between the two textile layers. In an exemplary embodiment of the first aspect of the present invention, said predefined volumes or areas (which define the predefined positions) between the two textile layers, in which each of said plurality of weight elements are arranged, do not overlap. This is advantageous since it prevents the plurality of weight elements from colliding and creating noise.

The plurality of weight elements does not necessarily include each and every one of the weight elements comprised by the blanket, but may be a subgroup out of all of the weight elements. The blanket may comprise other weight elements which are not part of the plurality of weight elements as defined. Other weight elements may be arranged in a different way than the plurality of weight elements. However, none of said plurality of weight elements being in direct contact with another weight element should be interpreted as none of the plurality of weight elements being in direct contact with any other weight element, even though the other weight element may not be part of the plurality of weight elements.

By weight element is meant any element comprised by the blanket having the purpose of increasing the weight of the blanket.

According to the first aspect of the present invention, the therapeutic blanket comprises a plurality of closed compartments arranged between said first textile layer and said second textile layer, each one of said plurality of closed compartments contains only one of said plurality of weight elements. This is advantageous since it prevents the weight elements from colliding, which minimizes the noise created by the blanket.

By closed compartment is meant a compartment which is substantially closed. In other words, closed compartment should in this context be interpreted as a compartment from which the weight element which is contained in it cannot unintentionally fall out or be removed. The closed compartment does not have to be completely sealed. For example, if the compartments are closed by seams, any length of stitches which will prevent the weight element from exiting the compartment through the seam is conceivable.

The compartments may be formed by attaching the second textile layer to the first textile layer, around each weight element. The attachment between the layers may for example be achieved by sowing, gluing, welding or riveting.

Each one of said plurality of closed compartments containing one of said plurality of weight elements does not exclude the possibility of having additional compartments in the blanket not containing such weight elements. For example, in an exemplary embodiment at least one empty closed compartment may be arranged between said first textile layer and said second textile layer. In another exemplary embodiment, at least one closed compartment containing a weight element not comprised by said plurality of weight elements may be arranged between said first textile layer and said second textile layer. According to the first aspect of the invention, each of the plurality of closed compartments in the blanket contains only one of said plurality of weight elements.

Arranging only one single weight element in each compartment is advantageous since it minimises the noise generated when the blanket is moved. If two weight elements are placed within the same compartments, they may collide when the user moves or when the blanket is being moved, which may cause an unpleasant or undesirable noise. Having only one single weight element in each compartment may therefore improve user comfort.

According to an exemplary embodiment of the first aspect of the present invention, the area of each compartment corresponds to 200 - 1500 % of a footprint of the weight element arranged in said compartment, preferably 400 - 1300 %, most preferably 800 - 1200%. Having compartments that have a greater area than the footprint of the weight elements arranged in said compartments improves the breathability of the blanket.

By footprint of the weight element is meant a two-dimensional projection of the weight element onto the first textile layer, when the blanket is lying flat on a horizontal surface. In other words, a footprint of the weight element may be seen as the shadow created by the element on the first textile layer, when the blanket is lying flat on a horizontal surface and light is cast on the element in a vertical direction.

According to the first aspect of the present invention, each one of said plurality of weight elements has at least one aperture extending through the weight element. A weight element may for example have one aperture. Weight elements having two, three, four or more apertures are also conceivable. It is not required that all weight elements within the plurality of weight elements have the same number of apertures. In some exemplary embodiments of the present invention, when the blanket is lying flat on a horizontal surface, the aperture extends at least partially in a direction not parallel to the horizontal surface. This further enhances the breathability of the blanket.

According to an exemplary embodiment of the first aspect of the present invention, said first textile layer and second textile layer are fixed to each other via one of said at least one aperture of each weight element. In other words, at least one weight element of the plurality of weight elements may be fixed by attaching the first textile layer to the second textile layer, through at least one of said at least one aperture extending through the weight element. For example, the first textile layer may be sown to the second textile layer, through the at least one aperture of the weight element.

According to an exemplary embodiment of the first aspect of the present invention, a total area of the at least one aperture in each weight element corresponds to 50 - 1000 % of a footprint of the weight element, preferably 100 - 300 %. By total area is meant the sum of the respective areas of the at least one aperture. This range is desirable since the apertures should be large enough to allow the blanket to breathe as desired, while still allowing the weight elements to have a desired weight and hence therapeutic effect.

According to an exemplary embodiment of the first aspect of the present invention, at least one of the weight elements is a ring shaped weight element. It should be noted that ring shaped does not in any way imply circular. By ring shaped element is meant an element which at least has one portion having the shape of a closed loop. The ring shaped elements may for example be circular, oval, square or polygonal.

According to an exemplary embodiment of the first aspect of the present invention, at least one of said weight elements comprises a ring shaped border having a central axis and a height; and a structure having a central portion and at least two leg portions extending from said central portion to separate points on said ring shaped border. Such an embodiment should in this application also be included in the term ring shaped weight element. According to another exemplary embodiment, said central portion is arranged along said central axis of said ring shaped border. This embodiment should in this application also be included in the term ring shaped weight element. According to yet another exemplary embodiment, said central portion is aligned with or extends above said height of the ring shaped border. This embodiment should in this application also be included in the term ring shaped weight element. Having a central portion is advantageous since it increases the number points in which the blanket may exert a pressure on the user.

The height of the ring shaped border (or the whole weight element) is defined as the thickness of the ring shaped border (or the whole weight element) measured along a direction parallel to a central axis. In other words, the height is defined as the vertical extension of the ring shaped border (or the whole weight element) when the weight element lies on a horizontal surface.

According to an exemplary embodiment of the first aspect of the present invention, the weight elements have a height of between 2 - 15 mm, preferably 5 - 10 mm. In one exemplary embodiment, the weight elements have a height of 7 mm.

According to an exemplary embodiment of the first aspect of the present invention, each one of said plurality of weight elements has a weight, and at least one of said weight elements has a weight which is different from the rest of the weight elements. This is advantageous since it may be desired to subject different parts of the body to different pressure. For example, for user comfort it may be advantageous to put less pressure on the user's feet, since it may be uncomfortable with too much pressure when the user is lying on his/her back. For this reason, the weight elements arranged in the portion of the blanket which is supposed to cover the user's feet may have a lighter weight than the rest of plurality of weight elements.

According to an exemplary embodiment of the first aspect of the present invention, the therapeutic blanket further comprises a layer of padding. According to another exemplary embodiment, said layer of padding is arranged between said second textile layer and a third textile layer. This is advantageous since it enhances user comfort.

According to an exemplary embodiment of the first aspect of the present invention, each weight element is preferably made in one piece. In other words, a weight element in the present context is not to be construed as a container holding a plurality of weight elements but as a single piece (e.g. a ring, a cross, or the like). Containers acting as weight elements are loud and provide inadequate air flow/ventilation through the blanket. Neither is a chain or the like to be construed as a weight element in the present context, but rather a plurality of weight elements that are linked to each other.

According to an exemplary embodiment of the first aspect of the present invention, the weight elements are made of a metallic, plastic, mineral or ceramic material, or a mix thereof. This is advantageous since there are metallic/plastic/ceramic materials that can provide the desired weight without being too bulky, while at the same time being strong enough to stand daily use and washing of the blanket. According to one exemplary embodiments, all weight elements within the plurality of weight elements may be made of the same material. This is advantageous since it simplifies the production of the blanket. According to another exemplary embodiment, at least one of said plurality of weight elements is made of a material which is different from the rest of the weight elements. This may be advantageous since it is a way to allow all weight elements within the plurality of weight elements to have the same dimensions, even though they may have different weights.

According to an exemplary embodiment of the first aspect of the present invention, at least one of the textile layers comprises flame retardant material. For example, it may be made of a material called Trevira® CS. This is desirable since the blankets can be used in psychiatric care, where flame retardant blankets may be required. According to another exemplary embodiment, said layer of padding may also be made of a flame retardant material. In other embodiments, the textile layer(s) may be made of any other suitable textile material. This may be advantageous since it may reduce the cost of the blanket.

According to an exemplary embodiment of the first aspect of the present invention, the therapeutic blanket may have a rectangular shape. This may for example be suitable for use by a human. According to another exemplary embodiment, the blanket may have a shape adapted for lying on a horse back or the back of a dog. According to another exemplary embodiment, the blanket may be part of a sleeping bag. According to yet another exemplary embodiment, a blanket according to the present invention may be part of a toes muff or foot muff for use with a baby stroller.

For human use, the blanket may suitably have a weight of between 4-12 kg, depending on what is desired by the user. A weight outside of this span is also conceivable.

According to a second aspect of the present invention, a method for manufacturing a therapeutic blanket according to claim 11 s provided. The method comprises:
- placing a plurality of weight elements on a first textile layer in a row across a width of the first textile layer, and wherein the weight elements are positioned on the first textile layer such that the weight elements are not in direct contact with each other;
- placing a second textile layer over said row of weight elements; and
- attaching said second textile layer to said first textile layer by forming a closed compartment around each one of said plurality of weight elements such that each of said plurality of weight elements is individually fixed at a predefined position between said first textile layer and said second textile layer, and such that none of the weight elements out of the plurality of weight elements is in contact with another weight element and wherein each weight element out of the plurality of weight elements comprises an aperture extending through the weight element.

According to an exemplary embodiment of the second aspect of the present invention, said closed compartment around each one of said plurality of weight elements is formed such that a contour of each weight element is visible on said therapeutic blanket. By forming the closed compartments so that the contour of each weight element is visible on said therapeutic blanket, the weight elements are prevented from flipping inside the compartments. Further, having a rough surface with visible contours of the weight elements may enhance the therapeutic effect of the blanket.

According to an exemplary embodiment of the second aspect of the present invention, the method may be used to manufacture a therapeutic blanket according to the first aspect of the present invention. All of the exemplary embodiments and advantages described in relation to the first aspect of the invention are also applicable to the second aspect of the invention.

These and other features and advantages of the present invention will in the following be further clarified with reference to the appended drawings showing different embodiments of the present invention.

### BRIEF DESCRIPTION OF THE DRAWINGS

Figure 1 is an exploded view showing an exemplary embodiment of a therapeutic blanket according to the first aspect of the present invention.
Figure 2 is an exploded view of an exemplary embodiment of the first textile layer and two weight elements according to the first aspect of the present invention.
Figure 3 is a top view of the same exemplary embodiment of a therapeutic blanket according to the first aspect of the present invention as is illustrated in Figure 1.
Figure 4 is a top view of an exemplary embodiment of a therapeutic blanket according to the first aspect of the present invention.
Figure 5a - 5c are partial top views of exemplary embodiments of therapeutic blankets according to the first aspect of the present invention.
Figure 7a - 16a are top views of exemplary embodiments of weight elements for use in a therapeutic blanket according to the first aspect of the present invention.
Figure 7b - 16b are perspective views of the same exemplary embodiments of weight elements as are illustrated in Figure 7a - 16a.
Figure 17 is a flow chart of an exemplary embodiment of a method for manufacturing a therapeutic blanket according to the second aspect of the present invention.

### DETAILED DESCRIPTION OF EMBODIMENTS

In the following detailed description, some embodiments of the present invention will be described. However, it is to be understood that features of the different embodiments are exchangeable between the embodiments and may be combined in different ways, unless anything else is specifically indicated. Even though in the following description, numerous specific details are set forth to provide a more thorough understanding of the present invention, it will be apparent to one skilled in the art that the present invention may be practiced without these specific details. In other instances, well known constructions or functions are not described in detail, so as not to obscure the present invention.

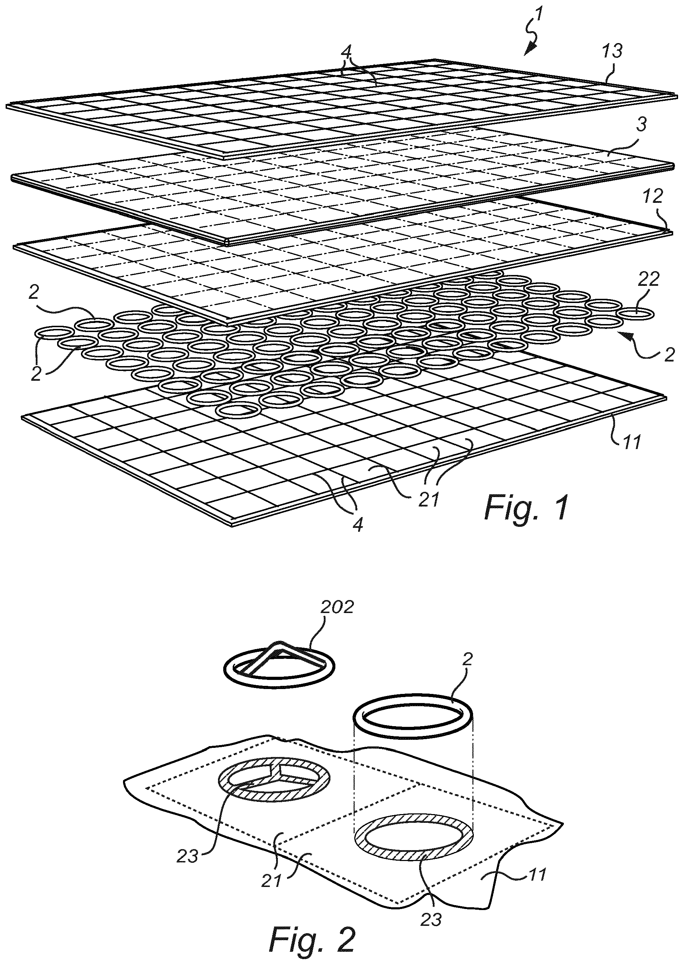

Figure 1 is an exploded view showing an exemplary embodiment of a therapeutic blanket according to the first aspect of the present invention. The blanket 1 comprises a first textile layer 11, a second textile layer 12 and a third textile layer 13. The textile layers 11, 12, 13 may all be made of a flame retardant material. Alternatively, only one or two of the textile layers may be made of a flame retardant material, or none of them may be made of a flame retardant material. An example of a suitable flame retardant material is Trevira® CS.

The illustrated blanket 1 further comprises a plurality of weight elements 2 arranged between the first textile layer 11 and the second textile layer 12. Each one of the plurality of weight elements 2 is individually fixed at a predefined position between the first textile layer 11 and the second textile layer 12, and none of the plurality of weight elements 2 are in direct contact with another weight element 2. The purpose of having a plurality of weight elements 2 is to increase the weight of the therapeutic blanket 1, to achieve a therapeutic effect. Suitable materials for the plurality of weight elements 2 may be metallic or plastic materials, or a mix thereof. In some exemplary embodiments, the weight elements 2 is suitably made of stainless steel or aluminium.

The weight elements 2 in the illustrated exemplary embodiment are ring shaped and have an aperture 22 extending through the weight element. The weight elements 2 may be formed by a wire, such as a metallic wire. This wire may have a diameter of 2-9 mm, preferably 3-5 mm. In the illustrated exemplary embodiment, the wire may have a diameter of 4 mm. The outer diameter of a ring shaped weight element 2 may suitably be 15-70 mm, preferably 30-50 mm. In the illustrated exemplary embodiment, the outer diameter of the ring shaped weight elements may be 40 mm. In the exemplary embodiment where the weight elements have a circular ring shape, an outer diameter of 40 mm and a wire diameter of 4 mm, the weight element has a footprint of 452 mm². In other exemplary embodiments, the footprint may have an area of between 80 - 2000 mm². The term "footprint" is further elucidated in reference to Fig. 2

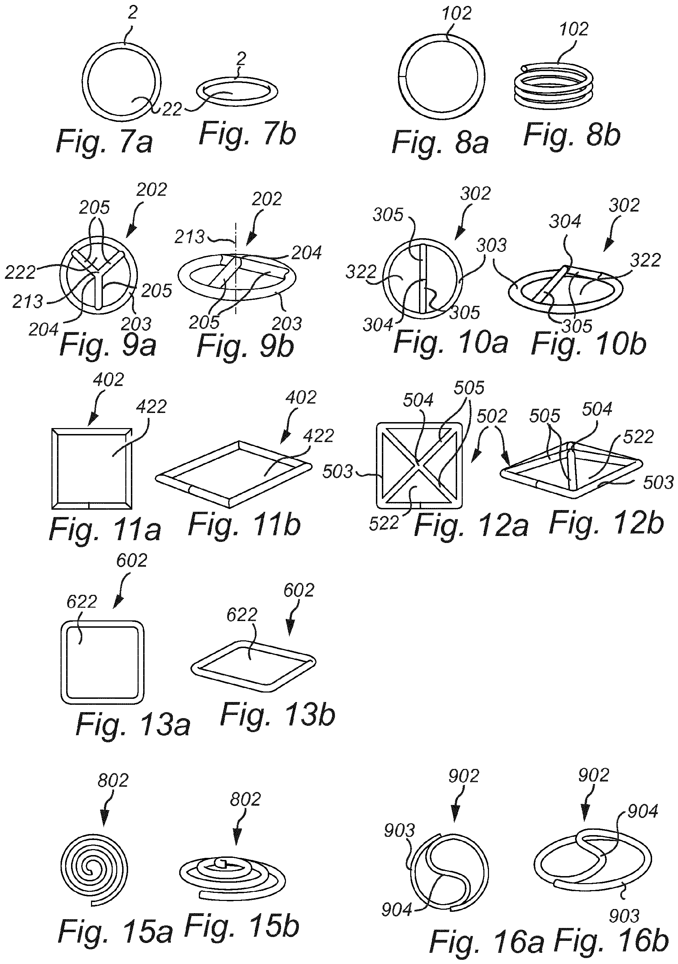

The aperture 22 of the weight element 2 may have a diameter of 10 - 65 mm. Other aperture diameters are also conceivable. It is also conceivable that the aperture(s) is/are not circular. Different types of apertures will be described more in relation to figures 7 - 16. In the illustrated embodiment in Figure 1, the aperture 22 has a diameter of 32 mm. This means that the aperture has an area of 804 mm². In this particular exemplary embodiment, the total area of the at least one aperture in each weight element corresponds to 178% of the footprint of the weight element.

The weight elements 2 may have a height of between 2 - 15 mm, preferably 5 - 10 mm. In the illustrated exemplary embodiment, the height of the weight elements 2 corresponds to the diameter of the thread. In other exemplary embodiments, the weight elements may have a height which is different from the diameter of the thread. This will be further described in relation to figures 7 - 16.

In the illustrated embodiment, the blanket 1 also comprises a plurality of closed compartments 21 arranged between the first textile layer 11 and the second textile layer 12, each one of the plurality of closed compartments 21 contains only one of the plurality of weight elements 2. The plurality of closed compartments 21 arranged between the first textile layer 11 and the second textile layer 12 may be defined by seams 4. In the illustrated exemplary embodiment, the closed compartments 21 have a square shape, but other shapes are also conceivable. The closed compartments 21 may have a width/height/diameter/mean diameter (depending on shape) of 30 - 100 mm. In the illustrated embodiment, the compartments 21 have a width and height of 70 mm, which means that they have an area of 4900 mm². The area of each compartment thus corresponds to 1084% of the footprint of the weight element 2 arranged in said compartment. In comparison, if the ratio between the area of each compartment and the footprint of the weight element arranged in said compartment would be 100%, the blanket would have very poor breathing properties. A higher ratio allows for a better air flow through the blanket. On the other hand, a too high ratio would indicate that the contact area between the weight elements and the user is very small, which would result in a reduced therapeutic effect.

The illustrated blanket further comprises a layer of padding 3, arranged between the second textile layer 12 and the third textile layer 13. This layer of padding 3 may be made of any suitable material, suitably padding material of the types used in conventional blankets. If the blanket is to be used in psychiatric care, the layer of padding may suitably be made of a flame retardant material.

The blanket 1 in the illustrated embodiment has a rectangular shape, which is suitable for use by a human. When using the blanket 1 for a horse or any other animal, the blanket may have a different shape. The shape of the blanket 1 is not limited to the illustrated embodiment, and should not be considered as an essential feature of the invention. Neither should the illustration be considered as made to scale in terms of the relation between the sizes of the different components. In the illustrated embodiment, there are 8x12 weight elements 2. This is however only to be seen as an illustrative example. A blanket for human use may for example comprise 150 - 500 weight elements. Other numbers of weight elements are also conceivable. In some embodiments comprising closed compartments, the number of closed compartments may be greater than the number of weight elements in the blanket.

Figure 2 is an exploded view of an exemplary embodiment of the first textile layer 11 and two weight elements 2, 202 according the first aspect of the present invention. Two different type of weight elements 2, 202 are shown, as well as their footprints 23 and the area of their respective compartments 21. The area of the compartment 21 in which the weight element 2, 202 is arranged must be greater than the area of the footprint 23 of the weight element. Preferably, the area of the compartment 21 should be significantly greater, since this enhances the breathability of the blanket.

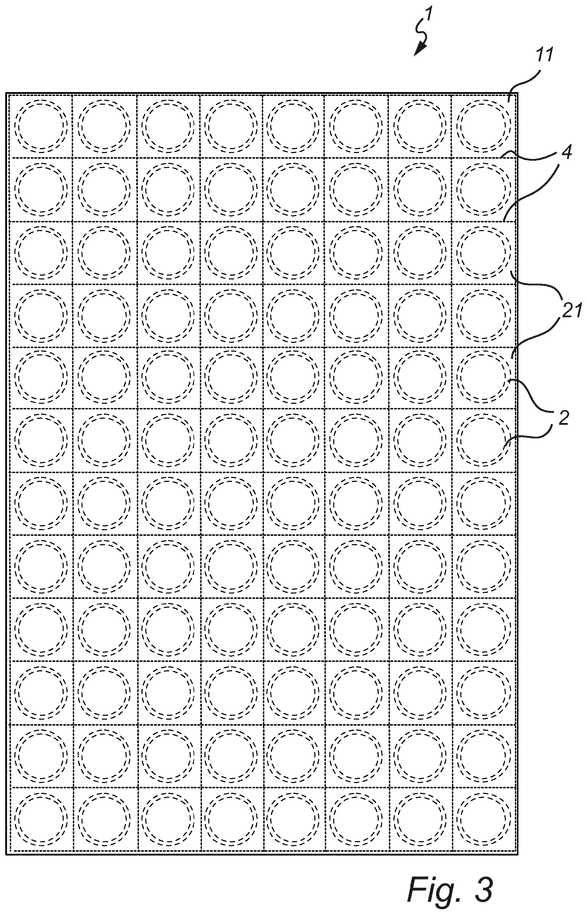

Figure 3 is a top view of the same exemplary embodiment of a therapeutic blanket 1 as is illustrated in Figure 1. From this view, the first textile layer 11 and the seams 4 defining the plurality of compartments 21 are visible. The contours of the plurality of weight elements 2 are also visible on the first textile layer 11.

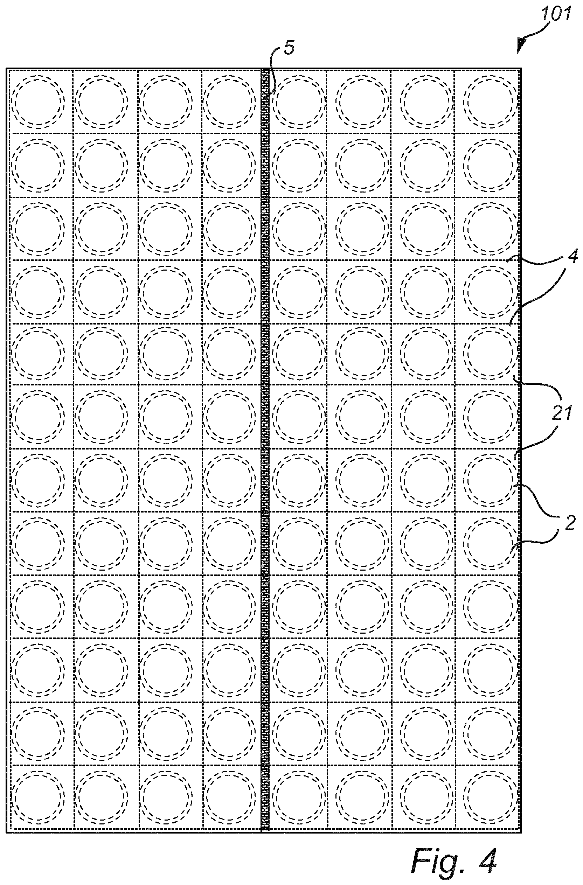

Figure 4 is a top view of an exemplary embodiment of a therapeutic blanket according to the first aspect of the present invention. In this exemplary embodiment, the blanket 101 further comprises a zipper 5, allowing the blanket 101 to be split in two parts. This is beneficial for blankets having a weight higher than what regular washing machines are capable of washing. By being able to split the blanket in two parts, the two parts can be washed separately without exceeding the maximum weight for the washing machine.

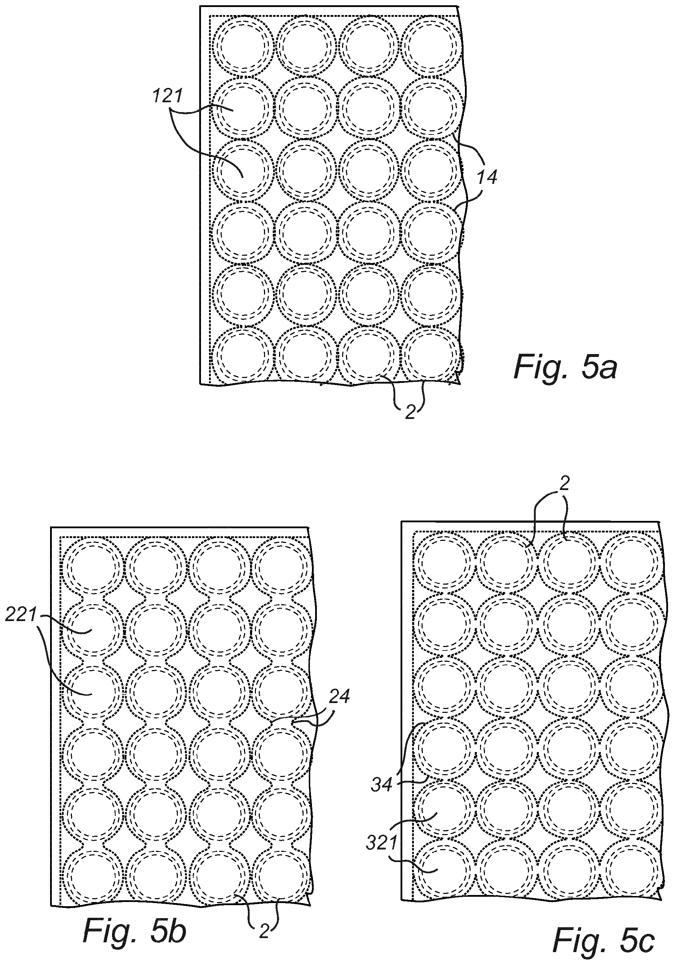

Figure 5a - 5c are partial top views of exemplary embodiments of therapeutic blankets according to the first aspect of the present invention. These exemplary embodiments may have the same features as the embodiment illustrated in Figure 1 and 3, but in these embodiments the compartments 121, 221, 321 have different shapes. The closed compartments 121, 221, 321 are in these examples defined by different types of seams 14, 24, 34 having a curved extension. This may be preferred since it may reduce the number of seams needed, or in other ways simplify the manufacturing process.

Figure 7a - 16a are top views of exemplary embodiments of weight elements for use in a therapeutic blanket according to the first aspect of the present invention. Figure 7b - 16b are perspective views of the same exemplary embodiments of weight elements as are illustrated in Figure 7a - 16a.

Figures 7a and 7b show the same embodiment of a weight element 2 as is comprised by the blankets illustrated in Figures 1 and 3-5. This weight element 2 is circularly ring shaped and has an aperture 22 extending through the weight element. This embodiment may be preferred since it is relatively inexpensive to manufacture.

Figure 8a and 8b show another embodiment of a weight element 102. This embodiment is also a circularly ring shaped weight element 102, however in this embodiment the thread used to manufacture the weight element 102 has been winded several turns, to create several layers. An advantage of this type of weight element is that it allows for having a greater height of the weight element, without having to increase the diameter of the thread. A greater height may induce an improved therapeutic effect, while an increase in thread diameter would reduce the breathability of the blanket. Alternatively, a similar effect may be achieved by stacking two or more of the same weight elements on top of each other and fixing them together permanently thereby forming a single weight element with increased height.

Figure 9a and 9b show another embodiment of a weight element 202. This embodiment comprises a ring shaped border 203 having a central axis 213 and a height, and a structure having a central portion 204 and three leg portions 205 extending from the central portion 204 to separate points on the ring shaped border 203. The central portion 204 is in this embodiment arranged along the central axis 213 of the ring shaped border 203, and the central portion 204 extends above the height of the ring shaped border 203. The weight element 202 has three apertures 222. This embodiment may be preferred since it includes an extra point of contact between the weight element and the user, and the central portion 204 extending above the height of the ring shaped border 203 may further enhance the therapeutic effect due to the extra pressure caused by the central portion 204.

Figure 10a and 10b show an embodiment of a weight element 302 which is similar to the embodiment shown in Figure 9a and 9b. However, in the embodiment shown in Figure 10a and 10b, the structure with the central portion 304 only has two leg portions 305 extending from the central portion 304 to separate points on the ring shaped border 303. The weight element has two apertures 322. This embodiment may be preferred since it has a smaller footprint than the embodiment in Figure 9a and 9b, which increases the breathability of the blanket.

Figure 11a and 11b show another embodiment of a weight element 402, similar to the embodiment shown in Figure 7a and 7b. The weight element in Figure 11a and 11b is also ring shaped, however instead of circular, it has a square shape. Like the embodiment in Figure 7a and 7b, this embodiment has one aperture 422.

Figure 12a and 12b show another embodiment of a weight element 502, having a square ring shaped border 503 and a structure having a central portion 504 and four leg portions 505 extending from the central portion 504 to separate points on the ring shaped border 503. This embodiment has four apertures 522.

Figure 13a and 13b show an embodiment of a weight element 602 which is similar to the embodiment shown in Figure 11a and 11b. This embodiment also shows a weight element 602with a square ring shape and one aperture 622. However, in this embodiment, the outer corners of the square are slightly rounded, which may improve user comfort.

Figure 15a and 15b show another exemplary embodiment of a weight element 802, having a spiral shape.

Figure 16a and 16b show another exemplary embodiment of a weight element 902, having a ring shaped border 903 and a central portion 904 extending above the height of the ring shaped border 903.

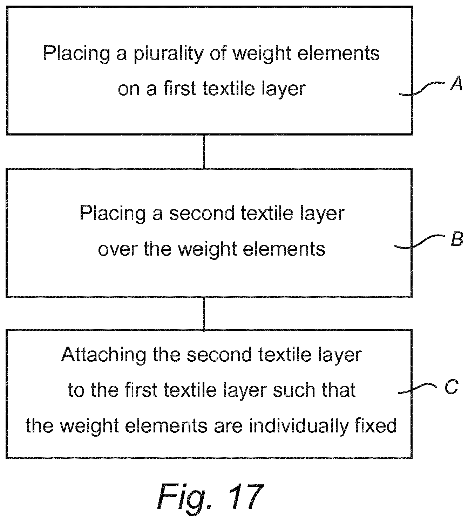

Figure 17 is a flow chart of an exemplary embodiment of a method for manufacturing a therapeutic blanket according to the second aspect of the present invention. The method may be used to manufacture any of the blankets described in relation to Figures 1-16, or any other embodiment of a blanket according to the first aspect of the present invention. The exemplary embodiment of the method comprises three steps. The first step A comprises placing a plurality of weight elements each comprising an aperture extending through the weight element on a first textile layer in a row across a width of the first textile layer, and wherein the weight elements are positioned on the first textile layer such that the weight elements are not in direct contact with each other. The second step B comprises placing a second textile layer over the row of weight elements. The third step C comprises attaching the second textile layer to the first textile layer such that each of the plurality of weight elements is individually fixed at a predefined position between the first textile layer and the second textile layer. The attachment should be made such that none of the weight elements out of the plurality of weight elements is in contact with another weight element.

The step of attaching the second textile layer to the first textile layer C comprises forming a closed compartment around each one of the plurality of weight elements preferably such that a contour of each weight element is visible on the therapeutic blanket. This method may for example be used to manufacture a blanket according to the exemplary embodiment illustrated in Figure 3.

The person skilled in the art realizes that the present invention by no means is limited to the embodiments described above. The features of the described embodiments may be combined in different ways, and many modifications and variations are possible within the scope of the appended claims. For example, weight elements of different shape, size and weight may be used in the same blanket. Blankets may comprise closed compartments which do not contain weight elements, and a combination of closed compartments and fixation points through apertures of the weight elements may be used. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements.

## Claims

1. A weighted therapeutic blanket comprising:
a first textile layer (11, 111);
a second textile layer (12, 112);
a plurality of weight elements (2) arranged between said first textile layer (11, 111) and said second textile layer (11, 111), and adapted to exert a pressure on the user, and
a plurality of closed compartments (21, 121, 221, 321) arranged between said first textile layer (11) and said second textile layer (12), each one of said plurality of closed compartments contains only one of said plurality of weight elements (2) so that
each one of said plurality of weight elements (2) is individually fixed at a predefined position between said first textile layer (11, 111) and said second textile layer (12, 112), wherein none of said plurality of weight elements (2) are in direct contact with another weight element, and wherein each one of said plurality of weight elements (2) has at least one aperture (22) extending through the weight element.

2. The weighted therapeutic blanket according to claim 1, wherein the area of each closed compartment (21, 121, 221, 321) corresponds to 200 - 1500 % of a footprint (23) of the weight element (2) arranged in said compartment, preferably 400 - 1300 %, most preferably 800 - 1200%.

3. The weighted therapeutic blanket according to claim 1, wherein said first textile layer (11, 111) and second textile layer (12, 112) are fixed to each other (6) via one of said at least one aperture (22) of each weight element (2).

4. The weighted therapeutic blanket according to claim 3, wherein a total area of the at least one aperture (22) in each weight element (2) corresponds to 50 - 1000 % of a footprint (23) of the weight element, preferably 100 - 300 %.

5. The weighted therapeutic blanket according to any one of the preceding claims, wherein at least one of the weight elements (2) is a ring shaped weight element.

6. The weighted therapeutic blanket according to any one of the preceding claims, wherein at least one of said weight elements (202, 302, 502) comprises:
a ring shaped border (203, 303, 503) having a central axis (213) and a height;
a structure having a central portion (204, 304, 504) and at least two leg portions (205, 305, 505) extending from said central portion (204, 304, 504) to separate points on said ring shaped border (203, 303, 503).

7. The weighted therapeutic blanket according to claim 6, wherein said central portion (204, 304, 504) is arranged along said central axis (213) of said ring shaped border (203, 303, 503).

8. The weighted therapeutic blanket according to any one of claims 6 - 7, wherein said central portion (204, 304, 504) is aligned with or extends above said height of the ring shaped border (203, 303, 503).

9. The weighted therapeutic blanket according to any one of the preceding claims, wherein the weight elements (2) have a height of between 2 - 15 mm, preferably 5 - 10 mm.

10. The weighted therapeutic blanket according to any one of the preceding claims, wherein each one of said plurality of weight elements (2) has a weight, and at least one of said weight elements has a weight which is different from the rest of the weight elements.

11. A method for manufacturing a weighted therapeutic blanket comprising:
placing a plurality of weight elements (2) on a first textile (11, 111) layer in a row across a width of the first textile layer, and wherein the weight elements (2) are positioned on the first textile layer (11, 111) such that the weight elements (2) are not in direct contact with each other;
placing a second textile layer (12, 112) over said row of weight elements; and
attaching said second textile layer (12, 112) to said first textile layer (11, 111) by forming a closed compartment (21, 121, 221, 321) around each one of said plurality of weight elements (2) such that each of said plurality of weight elements (2) is individually fixed at a predefined position between said first textile layer (11, 111) and said second textile layer (12, 112), such that none of the weight elements out of the plurality of weight elements (2) is in contact with another weight element, and wherein each weight element out of the plurality of weight elements (2) comprises an aperture (22) extending through the weight element.

12. The method according to claim 11, wherein said closed compartment (21, 121, 221, 321) around each one of said plurality of weight elements (2) is formed such that a contour of each weight element is visible on said therapeutic blanket (1).

## Patentansprüche

1. Beschwerte therapeutische Decke, umfassend:
eine erste Textilschicht (11, 111);
eine zweite Textilschicht (12, 112);
eine Vielzahl an Gewichtselementen (2), die zwischen der ersten Textilschicht (11, 111) und der zweiten Textilschicht (11, 111) angeordnet sind und angepasst sind um einen Druck auf den Benutzer auszuüben; und
eine Vielzahl an geschlossenen Kammern (21, 121, 221, 321) zwischen der ersten Textilschicht (11) und der zweiten Textilschicht (12) angeordnet sind, jede der Vielzahl an geschlossenen Kammern enthält nur eines der Vielzahl an Gewichtselementen (2), sodass
jedes der Vielzahl an Gewichtselementen (2) einzeln an einer vordefinierten Position zwischen der ersten Textilschicht (11, 111) und der zweiten Textilschicht (12, 112) fixiert ist, wobei keines der Vielzahl an Gewichtselementen (2) in direktem Kontakt mit einem anderen Gewichtselement steht, und wobei jedes der Vielzahl an Gewichtselementen (2) mindestens eine Öffnung (22) hat, die sich durch das Gewichtselement erstreckt.

2. Beschwerte therapeutische Decke nach Anspruch 1, wobei die Fläche jeder geschlossenen Kammer (21, 121, 221, 321) 200 bis 1500% einer Grundfläche (23) des in der Kammer angeordneten Gewichtselements (2) entspricht, vorzugsweise 400 bis 1300%, am meisten bevorzugt 800 bis 1200%.

3. Beschwerte therapeutische Decke nach Anspruch 1, wobei die erste Textilschicht (11, 111) und die zweite Textilschicht (12, 112) über eine der mindestens eine Öffnung (22) jedes Gewichtselements (2) aneinander (6) befestigt sind.

4. Beschwerte therapeutische Decke nach Anspruch 3, wobei eine Gesamtfläche der mindestens einen Öffnung (22) in jedem Gewichtselement (2) 50-1000% einer Grundfläche (23) des Gewichtselements entspricht, vorzugsweise 100-300%.

5. Beschwerte therapeutische Decke nach einem der vorhergehenden Ansprüche, wobei mindestens eines der Gewichtselemente (2) ein ringförmiges Gewichtselement ist.

6. Beschwerte therapeutische Decke nach einem der vorhergehenden Ansprüche, wobei mindestens eines der Gewichtselemente (202, 302, 502) umfasst:
eine ringförmige Grenze (203, 303, 503) mit einer zentralen Achse (213) und
einer Höhe;
eine Struktur mit einem zentralen Abschnitt (204, 304, 504) und mindestens zwei Beinabschnitten (205, 305, 505), die sich von dem zentralen Abschnitt (204, 304, 504) zu getrennten Punkten an der ringförmigen Grenze (203, 303, 503) erstrecken.

7. Beschwerte therapeutische Decke nach Anspruch 6, wobei der zentrale Abschnitt (204, 304, 504) und entlang der zentralen Achse (213) der ringförmigen Grenze (203, 303, 503) angeordnet ist.

8. Beschwerte therapeutische Decke nach einem der Ansprüche 6 bis 7, wobei der zentrale Abschnitt (204, 304, 504) mit der Höhe der ringförmigen Grenze (203, 303, 503) ausgerichtet ist oder sich über diese erstreckt.

9. Beschwerte therapeutische Decke nach einem der vorhergehenden Ansprüche, wobei die Gewichtselemente (2) eine Höhe zwischen 2 bis 15 mm, vorzugsweise 5 bis 10 mm aufweisen.

10. Beschwerte therapeutische Decke nach einem der vorhergehenden Ansprüche, wobei jedes der Vielzahl an Gewichtselementen (2) ein Gewicht aufweist und mindestens eines der Gewichtselemente ein Gewicht aufweist, das sich von den übrigen Gewichtselementen unterscheidet.

11. Verfahren zur Herstellung einer beschwerten therapeutischen Decke, umfassend:
Platzieren einer Vielzahl von Gewichtselementen (2) auf einer ersten Textilschicht (11, 111) in einer Reihe über eine Breite der ersten Textilschicht und wobei die Gewichtselemente (2) auf der ersten Textilschicht (11, 111) so positioniert sind, dass die Gewichtselemente (2) nicht in direktem Kontakt miteinander sind;
Platzieren einer zweiten Textilschicht (12, 112) über der Reihe von Gewichtselementen; und
Anbringen der zweiten Textilschicht (12, 112) an der ersten Textilschicht (11, 111) durch Bildung einer geschlossenen Kammer (21, 121, 221, 321) um jedes der Vielzahl an Gewichtselementen (2), so dass jedes der Vielzahl an Gewichtselementen (2) einzeln an einer vordefinierten Position zwischen der ersten Textilschicht (11, 111) und der zweiten Textilschicht (12, 112) fixiert ist, so dass keines der Gewichtselemente aus der Vielzahl an Gewichtselementen (2) in Kontakt mit einem anderen Gewichtselement ist, und wobei jedes Gewichtselement aus der Vielzahl an Gewichtselementen (2) eine Öffnung (22), die sich durch das Gewichtselement erstreckt, umfasst.

12. Verfahren nach Anspruch 11, wobei die geschlossene Kammer (21, 121, 221, 321) um jedes der Vielzahl an Gewichtselementen (2) so ausgebildet ist, dass eine Kontur jedes Gewichtselements auf der therapeutischen Decke (1) sichtbar ist.

## Revendications

1. Couverture thérapeutique lestée comprenant :
une première couche textile (11, 111) ;
une seconde couche textile (12, 112) ;
une pluralité d'éléments de lestage (2) disposés entre ladite première couche textile (11, 111) et ladite seconde couche textile (11, 111), et adaptés pour exercer une pression sur l'utilisateur, et
une pluralité de compartiments fermés (21, 121, 221, 321) agencés entre ladite première couche textile (11) et ladite seconde couche textile (12), chacun de ladite pluralité de compartiments fermés ne contenant qu'un seul de ladite pluralité d'éléments de lestage (2) de sorte que
chacun de ladite pluralité d'éléments de lestage (2) est fixé individuellement à une position prédéfinie entre ladite première couche textile (11, 111) et ladite seconde couche textile (12, 112), dans laquelle aucun de ladite pluralité d'éléments de lestage (2) n'est en contact direct avec un autre élément de lestage, et dans laquelle chacun de ladite pluralité d'éléments de lestage (2) a au moins une ouverture (22) s'étendant à travers l'élément de lestage.

2. Couverture thérapeutique lestée selon la revendication 1, dans laquelle la surface de chaque compartiment fermé (21, 121, 221, 321) correspond à 200-1500 % d'une empreinte (23) de l'élément de lestage (2) disposé dans ledit compartiment, de préférence 400-1300 %, plus préférablement 800 à 1200 %.

3. Couverture thérapeutique lestée selon la revendication 1, dans laquelle ladite première couche textile (11, 111) et ladite seconde couche textile (12, 112) sont fixées l'une à l'autre (6) via l'une de ladite au moins une ouverture (22) de chaque élément de lestage (2).

4. Couverture thérapeutique lestée selon la revendication 3, dans laquelle une surface totale de l'au moins une ouverture (22) dans chaque élément de lestage (2) correspond à 50-1000 % d'une empreinte (23) de l'élément de lestage, de préférence 100-300 %.

5. Couverture thérapeutique lestée selon l'une quelconque des revendications précédentes, dans laquelle au moins l'un des éléments de lestage (2) est un élément de lestage en forme d'anneau.

6. Couverture thérapeutique lestée selon l'une quelconque des revendications précédentes, dans laquelle au moins l'un desdits éléments de lestage (202, 302, 502) comprend :
une bordure en forme d'anneau (203, 303, 503) ayant un axe central (213) et une hauteur ;
une structure ayant une partie centrale (204, 304, 504) et au moins deux parties de jambe (205, 305, 505) s'étendant depuis ladite partie centrale (204, 304, 504) vers des points séparés sur ladite bordure en forme d'anneau (203, 303, 503).

7. Couverture thérapeutique lestée selon la revendication 6, dans laquelle ladite partie centrale (204, 304, 504) est disposée le long dudit axe central (213) de ladite bordure en forme d'anneau (203, 303, 503).

8. Couverture thérapeutique lestée selon l'une quelconque des revendications 6 à 7, dans laquelle ladite partie centrale (204, 304, 504) est alignée avec ou s'étend au-dessus de ladite hauteur de la bordure en forme d'anneau (203, 303, 503).

9. Couverture thérapeutique lestée selon l'une quelconque des revendications précédentes, dans laquelle les éléments de lestage (2) ont une hauteur comprise entre 2 et 15 mm, de préférence entre 5 et 10 mm.

10. Couverture thérapeutique lestée selon l'une quelconque des revendications précédentes, dans laquelle chacun de ladite pluralité d'éléments de lestage (2) a un poids, et au moins l'un desdits éléments de lestage a un poids qui est différent du reste des éléments de lestage.

11. Procédé de fabrication d'une couverture thérapeutique lestée comprenant :
placer une pluralité d'éléments de lestage (2) sur une première couche textile (11, 111) dans une rangée sur une largeur de la première couche textile, et dans lequel les éléments de lestage (2) sont positionnés sur la première couche textile (11, 111) de telle sorte que les éléments de lestage (2) ne sont pas en contact direct les uns avec les autres ;
placer une seconde couche textile (12, 112) sur ladite rangée d'éléments de lestage ; et
fixer ladite seconde couche textile (12, 112) à ladite première couche textile (11, 111) en formant un compartiment fermé (21, 121, 221, 321) autour de chacun de ladite pluralité d'éléments de lestage (2) de sorte que chacun de ladite pluralité d'éléments de lestage (2) est fixée individuellement à une position prédéfinie entre ladite première couche textile (11, 111) et ladite seconde couche textile (12, 112), de sorte qu'aucun des éléments de lestage parmi la pluralité d'éléments de lestage (2) n'est en contact avec un autre élément de lestage, et dans lequel chaque élément de lestage parmi la pluralité d'éléments de lestage (2) comprend une ouverture (22) s'étendant à travers l'élément de lestage.

12. Procédé selon la revendication 11, dans lequel ledit compartiment fermé (21, 121, 221, 321) autour de chacun de ladite pluralité d'éléments de lestage (2) est formé de telle sorte qu'un contour de chaque élément de lestage est visible sur ladite couverture thérapeutique (1).
